(19) Europäisches Patentamt
European Patent Office
Office européen des brevets

(11) Numéro de publication : **0 277 052 B1**

# (12) FASCICULE DE BREVET EUROPEEN

(45) Date de publication du fascicule du brevet : **11.03.92 Bulletin 92/11**

(21) Numéro de dépôt : **88400035.7**

(22) Date de dépôt : **08.01.88**

(51) Int. Cl.[5] : **C07D 493/04,** C07D 307/93, // (C07D493/04, 311:00, 303:00)

(54) **Dérivés de la 4,4-diméthyl tétrahydropyr-2-one, leur procédé de préparation et leur application à la synthèse de produits pyréthrinoides.**

(30) Priorité : **09.01.87 FR 8700150**
**30.07.87 FR 8710792**

(43) Date de publication de la demande : **03.08.88 Bulletin 88/31**

(45) Mention de la délivrance du brevet : **11.03.92 Bulletin 92/11**

(84) Etats contractants désignés : **AT CH DE ES FR GB IT LI NL**

(56) Documents cités :
**GB-A- 2 052 479**
**JOURNAL OF THE AMERICAN CHEMICAL SOCIETY, tome 102, 1980, pages 5974-5976, American Chemical Society, Washington, US; T. KATSUKI et al.: "The first practical method for asymmetric epoxidation"**

(73) Titulaire : **ROUSSEL-UCLAF**
**35, Boulevard des Invalides**
**F-75007 Paris (FR)**

(72) Inventeur : **Tessier, Jean**
**30, Rue Jean Moulin**
**F-94300 Vincennes (FR)**
Inventeur : **Demoute, Jean-Pierre**
**249 bis, rue de Rosny**
**F-93100 Montreuil Sous Bois (FR)**

(74) Mandataire : **Bourgouin, André et al**
**Département des Brevets ROUSSEL UCLAF**
**111, route de Noisy B.P. no 9**
**F-93230 Romainville (FR)**

**EP 0 277 052 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés de la 4,4-diméthyl tétrahydropyr-2-one, leur procédé de préparation et leur application à la synthèse de produits pyréthrinoïdes.

On connaissait déjà un dérivé du bicyclohexane le 2-acétoxy 6,6-diméthyl 4-oxo 3-oxabicyclo [3.1.0] hexane, son procédé de préparation et son application à la préparation de pyréthrinoïdes (cf GB 2.052.479).

L'invention a pour objet à titre de produit chimique nouveau, sous ses différentes formes d, l et dl, le composé de formule (I) :

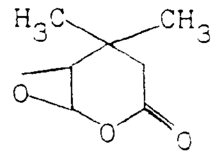

(I)

ainsi que les mélanges de ces différentes formes.

Les produits de formule (I) peuvent exister sous la forme d, l ou sous la forme dl.

Comme composé préféré de l'invention, on peut citer le composé de formule (I) sous la forme dl.

L'invention a également pour objet un procédé de préparation caractérisé en ce que l'on soumet à l'action d'un agent d'époxydation, choisi parmi les peracides comme l'acide peracétique, l'acide trifluoropéracétique, perphtalique, l'acide paranitroperbenzoïque, métachloroperbenzoïque, l'acide monopercamphorique, ou l'eau oxygénée en solution alcaline, ou l'hydropéroxyde d'hexafluoroacétone ou l'hydropéroxyde de terbutyle , ou les agents d'époxydation biochimiques avec une enzyme isolée ou par voie biochimique, le composé de formule (II) :

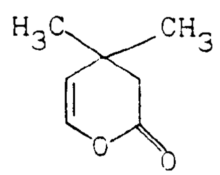

(II)

pour obtenir le composé de formule (I) que l'on sépare éventuellement en chacun de ses énantiomères.

L'invention a notamment pour objet un procédé de préparation, caractérisé en ce que l'agent d'époxydation est un peracide minéral ou organique, de l'eau oxygénée seule ou en présence d'un médiateur ou d'un oxydant capable de transférer un atome d'oxygène, il peut également s'agir d'un agent biochimique isolé (enzyme) ou au sein d'un milieu plus complexe (oxydation microbiologique).

Comme agent d'époxydation préféré, on peut citer l'acide métachloroperbenzoïque.

L'invention a également pour objet un procédé caractérisé en ce que l'on soumet le composé de formule (II) :

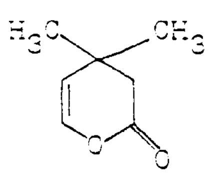

(II)

à l'action d'un agent d'époxydation tel que défini précédemment en présence d'un catalyseur métallique et d'un agent chiral pour obtenir un mélange non équimoléculaire de (5R,6S) époxy 4,4-di méthyl tétrahyropyr-2-one de formule ($I_A$) :

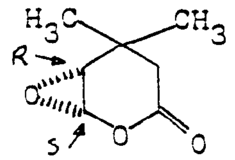

($I_A$)

et de son antipode optique de formule ($I_B$), à savoir le (5S,6R) époxy 4,4-diméthyl tétrahydropyr-2-one :

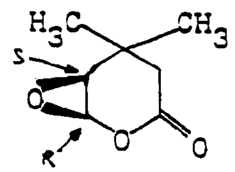

($I_B$)

Selon l'agent chiral, on obtient une majorité de produits ($I_A$) ou ($I_B$).

L'invention a plus particulièrement pour objet le procédé où le catalyseur métallique est le molybdène hexacarbonyle.

On peut également utiliser comme catalyseur métallique les dérivés du titane, du vanadium, du sélénium ou de l'osmium.

L'invention a tout sépcialement pour objet un procédé où l'agent chiral est le L(+) ou le D(-) tartrate de dialkyle dans lequel le radical alkyle renferme de 1 à 6 atomes de carbone.

On peut également citer comme agents chiraux, le L(+) ou le D(-) tartrate de diisopropyle ou encore le L(+) ou le D(-) tartrate d'éthyle.

Parmi les modes de réalisation préférés, on peut citer celui où l'époxydation a lieu en présence de L(+) tartrate de diéthyle en présence de molybdène hexacarbonyle.

Comme agent d'époxydation préféré, on peut citer d'hydrooéroxyde de terbutyle. On peut également utiliser d'autres agents d'époxydation choisis parmi ceux cités ci-dessus comme l'eau oxygénée ou les peracides.

Le procédé de l'invention ci-dessus décrit permet d'époxyder énantiosélectivement une double liaison ni conjuguée, ni hydroxylée en alpha.

Les produits de formule (I) trouvent leur application dans la synthèse de produits industriels, de formule (VI) :

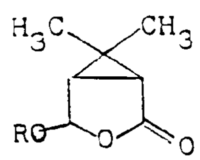

(VI)

décrits notamment dans le brevet français 1 580 474 et les brevets européens 0 023 454 et 0 024 241. L'invention a également pour objet un procédé de préparation des composés de formule VI

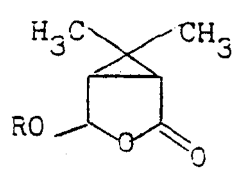

(VI)

dans laquelle R représente un radical méthyle, éthyle, n-propyle, isopropyle, cyclopropyle, n-butyle ou terbutyle, cyclobutyle, n-pentyle ou cyclopentyle, cyclohexyle ou menthyle, 1,1-diméthylallyle, éventuellement substitué par un ou plusieurs atomes d'halogènes, groupements OH ou SH, groupements OR′ ou SR′ dans lequel R′ représente un radical alcoyle renfermant de 1 à 8 atomes de carbone, groupements $NO_2$ ou

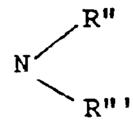

dans lequel R″ et R‴, identiques ou différents, représentent un atome d'hydrogène ou un radical alkyle renfermant de 1 à 8 atomes de carbone, groupements -C≡N ou $PO_4H_2$ ou groupements COalc′$_1$, $SO_2$alc′$_2$ ou $SO_3$alc′$_3$ dans lesquels alc′$_1$, alc′$_2$ ou alc′$_3$ représentent des radicaux de 1 à 18 atomes de carbone, ou R représente un radical $CH_2OCH_3$, $(CH_2)_2OCH_3$,

$CH_2CH_2OCH_2CH_3$, 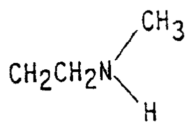 , 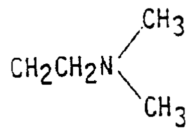 , 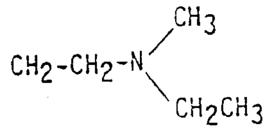 .

ou R représente un radical benzyle, ou R représente un radical phényle, caractérisé en ce que l'on soumet le composé de formule I défini précédemment à l'action d'un alcool de formule (III)

R-OH (II)

dans laquelle R est défini comme précédemment, pour obtenir le composé de formule (IV) correspondant :

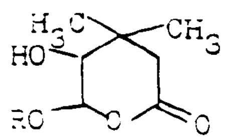

(IV)

que l'on soumet à l'action d'un composé de formule ZX dans laquelle X représente un atome d'halogène et Z représente un radical $R_1A$ dans lequel $R_1$ représente un radical alcoyle renfermant de 1 à 8 atomes de carbone ou un radical aryle éventuellement substitué et A représente un radical $-SO_2-$ ou un radical :

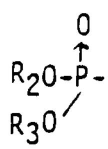

dans laquelle $R_2$ et $R_3$, identiques ou différents, représentent un radical alcoyle renfermant de 1 à 8 atomes de carbone, pour obtenir le composé de formule (V) :

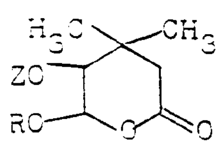

(V)

que l'on soumet à l'action d'un agent basique, pour obtenir le composé de formule (VI) :

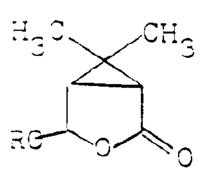

(VI)

R conservant la même signification que précédemment. il s'agit de préférence du radical $CH_2OCH_3$, $(CH_2)_2OCH_3$,

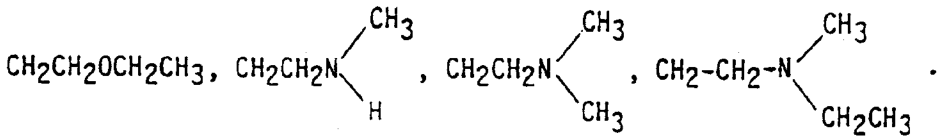

X représente de préférence un atome de chlore ou de brome.

Dans la définition de Z, $R_1$, $R_2$ et $R_3$ représentent de préference un radical méthyle, éthyle, n-propyle, isopropyle, terbutyle ou n-butyle, un radical phényle éventuellement substitué par un ou plusieurs radicaux alcoyles.

Comme agent basique auquel on soumet le composé de formule (Y), on peut utiliser les hydrures métalliques, les alcoolates alcalins ou alcalino-terreux, les dérivés organométalliques, les hydroxydes alcalins ou alcalino-terreux, les carbonates alcalins ou alcalino-terreux et les amides tertiaires.

Comme agents basiques préférés, on peut citer la soude ou la potasse.

L'invention a plus particulièrement pour objet l'application caractérisée en ce que

- le composé de formule (I) est racémique,
- le composé de formule (IV) est racémique avec les groupements RO et OH en position trans, R étant défini comme précédemment,
- le composé de formule (V) est racémique avec les groupements ZO et OR en position trans, Z étant défini comme précédemment et,
- le composé de formule (VI) obtenu est racémique.

L'invention a notamment pour objet l'application caractérisée en ce que R représente un radical méthyle, éthyle ou terbutyle ainsi que l'application caractérisée en ce que Z représente un radical $-SO_2CH_3$.

L'invention a également pour objet l'application caractérisée en ce que l'on prépare de façon énantiosélective la lactone de l'acide (1R,cis) 3,3-diméthyl 2-(dihydroxyméthyl) cyclopropane 1-carboxylique de formule :

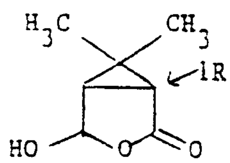

(Produit P)

appelée ci-après produit P, qui est utilisé dans la synthèse de pyréthtinoïdes biologiquement actifs (voir par exemple le brevet français 1 580 474).

La préparation du produit P peut être résumée par le schéma suivant :

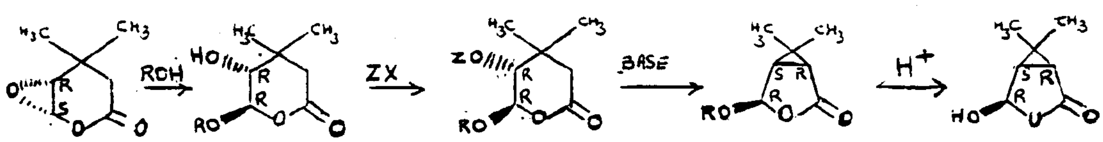


Les exemples suivants illustrent l'invention sans toutefois la limiter.

**Exemple 1 : 5,6-époxy 4,4-diméthyl tétrahydropyr-2-one racémique.**

On ajoute à 0°C 43,15 g d'acide métachloroperbenzoïque dans une solution de 25,2 g de 4,4-diméthyl 3,4-dihydro alpha-pyrone. On agite le mélange réactionnel pendant 3 heures, en laissant remonter la température à la température ambiante. On essore le précipité obtenu et l'empâte au chlorure de méthylène ; on lave le filtrat avec une solution de thiosulfate de sodium, puis avec une solution saturée de bicarbonate de sodium, enfin à l'eau. On sèche la phase organique et évapore à sec sous pression réduite. On récupère ainsi 28,5 g de produit brut. On purifie ce dernier par chromatographie sur silice, en éluant par un mélange d'hexane et d'éther isopropylique et obtient 23,08 g de produit recherché fondant vers 22°C.

Spectre RMN $CDCl_3$.

| | |
|---|---|
| Protons des méthyles géminés : | 1,13 - 1,26 ppm |
| Proton en 3 : | 2,03 à 2,75 ppm |
| Proton en 6 : | 5,22 - 5,27 ppm |
| autres protons (en 5) : | 2,98 à 3,05 ppm. |

**Exemple 2 : (5R,6S) époxy 4,4-diméthyl tétrahydropyr-2-one (produit $I_A$) et (5S,6R) époxy 4,4-diméthyl tétrahydropyr-2-one (produit ($I_B$).**

On refroidit à 0°C une solution renfermant 2,52 g de 4,4-diméthyl 3,4-dihydro alpha pyrone, 25 cm3 de toluène conservé sur siliporite, 4,12 g de L(+) tartrate de diéthyle et 110 mg de molybdène hexacarbonyle. On ajoute à 0°C, 25 cm3 d'une solution toluénique 2,9 M d'hydropéroxyde de terbutyle. On laisse le mélange réactionnel revenir à la température ambiante, et le maintient sous agitation pendant 20 jours. On verse le mélange réactionnel sur une solution de 10 g d'acide tartrique, 25 g de sulfate ferrique et 100 cm3 d'eau. On rince au toluène et agite pendant une heure entre 15°C et 20°C. On décante, extrait au toluène, lave à l'eau, sèche et distille à fin de solvant. On obtient une huile que l'on chromatographie en éluant par un mélange hexane-éther isopropylique (1-1). On isole le produit de rf = 0,15 et récupère, après évaporation du solvant, 436 mg d'un produit que l'on chromatographie à nouveau en éluant par le mélange hexane-acétate d'éthyle (85-15). On isole le produit de rf = 0,2, évapore les solvants sous vide et récupère ainsi 232 mg de produit recherché fondant à 22°C. $[alpha]_D = -33° \pm 2°$ (c = 0,6% toluène.

La proportion $\frac{IA}{IB}$ est de $\frac{3}{2}$.

**Exemple 3 :**

En opérant de façon identique à celle de l'exemple 2, avec le D(-) tartrate de diéthyle, on obtient un mélange $\frac{IA}{IB} = \frac{2}{3}$.

**Exemple 4 :**

En opérant de façon identique à celle de l'exemple 2, mais en remplaçant le L(+) tartrate de diéthyle par le L(+) tartrate de diisopropyle, on obtient un résultat similaire à celui de l'exemple 2.

Dans les exemples 2, 3 et 4, la pureté énantiomérique a été évaluée par RMN en présence de 20% de tris [3-(trifluorométhylhydroxyméthylène) (+) camphorate) Europium III EU $(tfc)_3$.

**FREQUENCES (ppm) dans $CDCl_3$.**

| | isomère 5R 6S | isomère 5S 6R |
|---|---|---|
| protons des méthyles | 1,28 et 1,38 | 1,28 et 1,42 |
| protons en 3 | 2,54 (d) et 2,92 (d) | |
| proton en 5 | 3,38 | 3,42 |
| proton en 6 | 5,71 | 5,59 (d) |

**Application 1 : 6-méthoxy 5-hydroxy 4,4-diméthyl tétrahydropyr-2-one dl.**

On ajoute à -15°C, une goutte d'acide paratoluène sulfonique dans une solution renfermant 1,42 g de 5,6-époxy 4,4-diméthyl tétrahydropyr-2-one (F ≃ 22°C), 14 cm3 de chlorure de méthylène et 320 mg de méthanol. On agite pendant 3 heures 30 minutes en laissant la température remonter à -10°C, on ajoute 0,4 cm3 de méthanol, on agite pendant 16 heures à -10°C, ajoute 40,4 cm3 de méthanol et un peu d'acide paratoluène sulfonique. On agite pendant 48 heures en laissant le milieu revenir à la température ambiante. On décante, lave avec une solution saturée de bicarbonate de sodium puis à l'eau, sèche et distille à sec sous pression réduite. On récupère ainsi 1,45 g de produit brut que l'on vhromatographie sur silice en éluant par le mélange hexane-acétate d'éthyle (1-1). on obtient ainsi 1,42 mg du produit recherché fondant à 72°C. Rf = 0,20.

Spectre RMN

H du $OCH_3$ = 3,6 ppm

Spectre RMN $CDCl_3$

Protons des méthyles géminés : 1,06 - 1,1 ppm
Protons en 32,2 - 2,4 ppm 2,5 - 2,77 ppm
Protons en 6 : 4,95 - 5,06 ppm
Protons en 5 : 3,4 - 3,5 ppm

**Application 2 : 6,6 diméthyl 4-(1-méthyléthoxy) 3-oxa bicyclo [3.1.0.] hexan-2-one.**

**Stade A :** 6-(1-méthyléthyloxy) 5-hydroxy 4,4-diméthyl tétrahydropyr-2-one dl.

On maintient sous agitation pendant 23 heures, à la température ambiante, 7 g de 5,6-époxy 4,4-diméthyl tétrahydropyr-2-one, 35 cm3 de 1-méthyl éthanol et 350 mg d'acide paratoluène sulfonique. On dilue à l'eau, extrait au chlorure de méthylène, lave avec une solution aqueuse saturée de bicarbonate de sodium, sèche et distille à sec sous pression réduite. On obtient ainsi 9,18 g de produit recherché brut que l'on purifie par chromatographie sur silice en éluant par le mélange hexane-acétate d'éthyle. On obtient ainsi 2,597 g de produit recherché fondant à 50°C.

Spectre RMN $CDCl_3$

Proton des méthyles : de 1,06 à 1,31 ppm
Proton du carbone 3 : 2,38 - 2,41 ppm
Proton du carbone 5 : 3,4 - 3,5 ppm
Proton du carbone 6 : 5,1 - 5,2 ppm.

**Stade B :** 6-(1-méthyléthoxy) 5-méthylsulfonyloxy 4,4-diméthyl tétrahydropyr-2-one.

On maintient sous agitation pendant 3 heures 1,556 g de 6-(1-méthyl éthoxy) 5-hydroxy 4,4-diméthyl tétrahydropyr-2-one, 7,5 cm3 de pyridine, 1 cm3 de chlorure de méthane sulfonyle. On dilue à l'acide chlorhydrique N, extrait au chlorure de méthylène, lave à l'eau, sèche et distille à sec sous pression réduite. On obtient ainsi 2,096 g de produit recherché fondant à 75°C.

Spectre RMN $CDCl_3$

Proton des méthyles géminés : 1,06 à 1,31 ppm
Proton du carbone 3 : 2,38 - 2,41 ppm
Proton du carbone 5 : 3,4 - 3,5 ppm
Proton du carbone 6 : 5,1 - 5,2 ppm
Protons des méthyles du radical méthyléthoxy : 1,23 et 1,27 ppm
Proton du carbone en 1 du radical 1-méthyléthoxy : 4,14 ppm.

**Stade C :** 6,6-diméthyl 4-(1-méthyléthoxy) 3-oxa bicyclo [3.1.0.] hexan-2-one, racémique.

On maintient sous agitation un mélange renfermant 1,94 g de 6-(1-méthyl éthoxy) 5-méthylsulfonyloxy 4,4-diméthyl tétrahydropyr-2-one, 10 volumes de chlorure de méthylène, 2,5 % en poids de chlorure de triéthylbutylammonium et 5 volumes de soude à 50%. L'évolution de la réaction est suivie en CCM. Une fois la réaction terminée, on dilue à l'eau salée, extrait au chlorure de méthylène et lave à l'eau salée. On sèche et distille à fin de solvant sous pression réduite. On chromatographie sur silice le résidu en éluant par le mélange hecane-acétate d'éthyle (1-1). onobtient ainsi 908 mg de produit recherché fondant vers 50°C.

Spectre RMN $CDCl_3$

Proton des méthyles géminés : 1,17 - 1,2 ppm
Protons en 1 et 5 de l'hexane : 2,02 ppm
Proton du carbone portant le radical 1-méthyléthoxy : 5,27 ppm
Proton du carbone en 1 de l'éthoxy : 3,82 à 4,21 ppm

**Application 3 : 6,6-diméthyl 4-(1,1-diméthyléthoxy) 3-oxa bicyclo [3.1.0] hexan-2-one.**

**Stade A :** 6-(1,1-diméthyléthoxy) 5-hydroxy 4,4-diméthyl tétrahydropyr-2-one.

On agite à la température ambiante pendant 41 heures, 500 mg de 5,6-époxy-4,4-diméthyl tétrahydropyr-2-one, 5 cm3 de 1,1-diméthyléthanol, 2,5 mg d'acide paratoluène sulfonique. On ajoute 1 cm3 de pyridine et distille sous pression réduite à fin de solvant. On obtient ainsi un produit que l'on utilise tel quel pour le stade suivant.

**Stade B :** 6-(1,1-diméthyléthoxy) 5-méthyl sulfonyloxy 4,4-diméthyl tétrahydropyr-2-one.

On ajoute au produit obtenu au stade A, 5 cm3 de pyridine et 1 cm3 de chlorure de méthane sulfonyle. On maintient le mélange réactionnel sous agitation pendant 3 heures 30 minutes. On dilue à l'acide chlorhydrique N, extrait au chlorure de méthylène, lave les extraits à l'eau. On sèche et distille le produit obtenu sous pression réduite. On obtient ainsi 677 mg de produit recherché sous forme brute, que l'on purifie par chromatographie sur silice en éluant par le mélange hexane-acétate d'éthyle (7-3). On obtient ainsi 410 mg de produit recherché

fondant à 64°C.

Spectre RMN $CDCl_3$

Proton des méthyles géminés : 1,06 à 1,20 ppm
Proton du carbone en 3 : 2,23 à 2,56 ppm
Proton du carbone en 6 : 5,58 ppm
Proton du carbone en 5 : 4,5 ppm
Protons du méthyle du radical $OSO_2CH_3$ : 3,13 ppm

**Stade C :** 6,6-diméthyl 4-(1,1-diméthyléthoxy) 3-oxabicyclo [3.1.0.] hexan-2-one.

En opérant comme pour la préparation du 6,6-diméthyl 4-(1-méthyléthoxy) 3-oxabicyclo [3.1.0.] hexan-2-one, à partir de 100 mg du composé 1,1-diméthyléthoxy correspondant, on obtient 52 mg du produit recherché fondant à 115°C.

Spectre RMN $CDCl_3$

Proton des méthyles géminés : 1,14 à 1,19 ppm
Protons en 1 et 3 du cyclopropane : 2,23 à 2,56 ppm
Protons du carbone portant le groupement 1,1-diméthyléthoxy : 5,36 ppm
Protons des méthyles du radical 1,1-diméthyléthoxy : 1,3 ppm.

## Revendications

Revendications pour l'Etat contractant: AT CH DE FR GB IT LI NL

1. A titre de produit chimique nouveau, sous ses différentes formes d, l et dl, le composé de formule (I) :

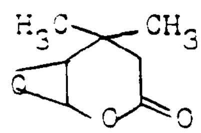

(I)

ainsi que les mélanges de ces différentes formes.

2. A titre de produit chimique nouveau, le composé de formule (I) sous sa forme dl.

3. Procédé de préparation du composé de formule (I) tel que défini à la revendication 1 ou 2, caractérisé en ce que l'on soumet à l'action d'un agent d'époxydation, choisi parmi les peracides comme l'acide peracétique, l'acide trifluoroperacétique, perphtalique, l'acide paranitroperbenzoïque, métachloroperbenzoïque, l'acide monopercamphorique, ou l'eau oxygénée en solution alcaline, ou l'hydropéroxyde d'hexafluoroacétone ou l'hydropéroxyde de terbutyle , ou les agents d'époxydation biochimiques avec une enzyme isolée ou par voie biochimique, le composé de formule (II) :

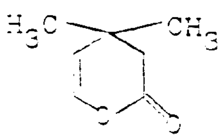

(II)

pour obtenir le composé de formule (I) que l'on sépare éventuellement en chacun de ses énantiomères.

4. Procédé de préparation selon la revendication 3, caractérisé en ce que l'agent d'époxydation est soit un peracide minéral ou organique, soit l'eau oxygénée seule ou en présence d'un médiateur ou d'un oxydant capable de transférer un atome d'oxygène, soit un agent biochimique isolé ou au sein d'un milieu plus complexe (oxydation microbiologique).

5. Procédé selon la revendication 4, caractérisé en ce que l'agent d'époxydation est l'acide métachloroperbenzoïque.

6. Procédé de préparation du composé de formule (I) tel que défini à la revendication 1, caractérisé en ce que l'on soumet le composé de formule (II) :

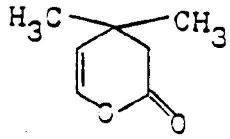

(II)

à l'action d'un agent d'époxydation tel que défini précédemment en présence d'un catalyseur métallique et d'un agent chiral pour obtenir un mélange non équimoléculaire de (5R,6S) époxy 4,4-diméthyl tetrahyropyr-2-one de formule ($I_A$) :

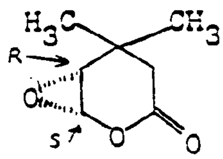

($I_A$)

et de son antipode optique de formule ($I_B$), à savoir le (5S,6R) époxy 4,4-diméthyl tétrahydropyr-2-one :

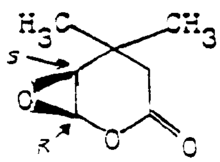

($I_B$)

7. Procédé de préparation selon la revendication 6, caractérisé en ce que le catalyseur métallique est le molybdène hexacarbonyle.

8. Procédé selon la revendication 6 ou 7, caractérisé en ce que l'agent chiral est le L(+) ou le D(-) tartrate de dialkyle.

9. Procédé selon la revendication 8, caractérisé en ce que l'époxydation a lieu en présence de L(+) tartrate de diéthyle en présence de molybdène hexacarbonyle.

10. Procédé selon l'une quelconque des revendications 6 à 9, caractérisé en ce que l'agent d'époxadation est l'hydropéroxyde de terbutyle.

11. Procédé de préparation des composés de formule VI

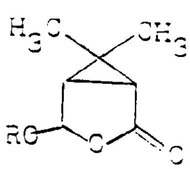

(VI)

dans laquelle R représente un radical méthyle, éthyle, n-propyle, isopropyle, cyclopropyle, n-butyle ou terbutyle, cyclobutyle, n-pentyle ou cyclopentyle, cyclohexyle ou menthyle, 1,1-diméthylallyle, éventuellement substitué par un ou plusieurs atomes d'halogènes, groupements OH ou SH, groupements OR′ ou SR′ dans lequel R′ représente un radical alcoyle renfermant de 1 à 8 atomes de carbone, groupements $NO_2$ ou

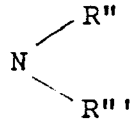

dans lequel R″ et R‴, identiques ou différents, représentent un atome d'hydrogène ou un radical allyle renfermant de 1 à 8 atomes de carbone, groupements -C≡N ou $PO_4H_2$ ou groupements COalc'$_1$, $SO_2$alc'$_2$ ou $SO_3$alc'$_3$ dans lesquels alc'$_1$, alc'$_2$ ou alc'$_3$ représentent des radicaux de 1 à 18 atomes de carbone, ou R représente un radical $CH_2OCH_3$, $(CH_2)_2OCH_3$,

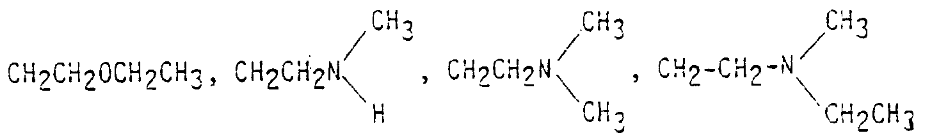

ou R représente un radical benzyle, ou R représente un radical phényle, caractérisé en ce que l'on soumet le composé de formule I défini à la revendication 1 à l'action d'un alcool de formule (III)

R-OH (III)

dans laquelle R est défini comme précédemment, pour obtenir le composé de formule (IV) correspondant :

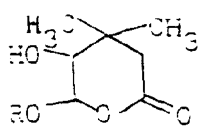

(IV)

que l'on soumet à l'action d'un composé de formule ZX dans laquelle X représente un atome d'halogène et Z représente un radical $R_1A$ dans lequel $R_1$ représente un radical alcoyle renfermant de 1 à 8 atomes de carbone ou un radical aryle éventuellement substitué et A représente un radical $-SO_2-$ ou un radical :

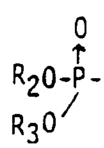

dans laquelle $R_2$ et $R_3$, identiques ou différents, représentent un radical alcoyle renfermant de 1 à 8 atomes de carbone, pour obtenir le composé de formule (V) :

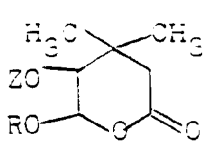

(V)

que l'on soumet à l'action d'un agent basique, pour obtenir le composé de formule (VI) :

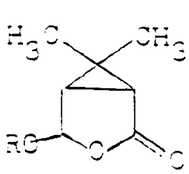

(VI)

R conservant la même signification que précédemment.

12. Application selon la revendication 11, caractérisée en ce que

- le composé de formule (I) est racémique,
- le composé de formule (IV) est racémique avec les groupements RO et OH en position trans, R étant défini comme précédemment,
- le composé de formule (V) est racémique avec les groupements ZO et OR en position trans, Z étant défini comme précédémment et,
- le composé de formule (VI) obtenu est racémique.

13. Application selon la revendication 12, caractérisée en ce que R représente un radical méthyle, éthyle ou terbutyle.

14. Application selon la revendication 12 ou 13, caractérisée en ce que Z représente un radical $-SO_2CH_3$.

**Revendications pour l'Etat contractant suivant: ES**

1. Procédé de préparation des composés de formule (VI) :

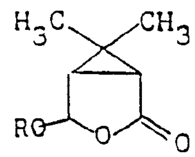

(VI)

sous formes racémique ou optiquement active ainsi que leur mélange dans laquelle R représente un radical méthyle, éthyle, n-propyle, isopropyle, cyclopropyle, n-butyle ou terbutyle, cyclobutyle, n-pentyle ou cyclopentyle, cyclohexyle ou menthyle, 1,1-diméthylallyle, éventuellement substitué par un ou plusieurs atomes d'halogènes, groupements OH ou SH, groupements OR′ ou SR′ dans lequel R′ représente un radical alcoyle renfermant de 1 à 8 atomes de carbone, groupements $NO_2$ ou

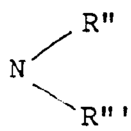

dans lequel R″ et R‴, identiques ou différents, représentent un atome d'hydrogène ou un radical alkyle renfermant de 1 à 8 atomes de carbone, groupements -C≡N ou $PO_4H_2$ ou groupements COalc′$_1$, $SO_2$alc′$_2$ ou $SO_3$alc′$_3$ dans lesquels alc′$_1$, alc′$_2$ ou alc′$_3$ représentent des radicaux de 1 à 18 atomes de carbone, ou R représente un radical $CH_2OCH_3$, $(CH_2)_2OCH_3$,

$CH_2CH_2OCH_2CH_3$, 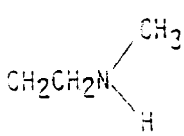 , 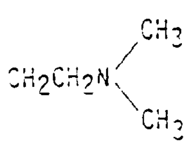 , 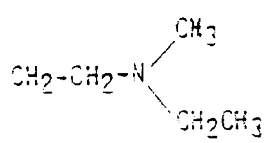

ou R représente un radical benzyle, ou R représente un radical phényle, caractérisé en ce que l'on soumet le composé de formule (II) :

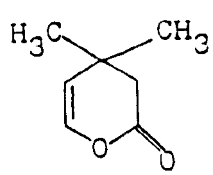

(II)

soit à l'action d'un agent d'epoxydation pour obtenir le composé de formule (I) :

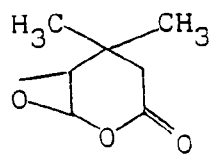

(I)

que l'on sépare éventuellement en chacun de ses énantiomères, soit à l'action d'un agent d'époxydation en présence d'un catalyseur métallique et d'un agent chiral pour obtenir un mélange non équimoléculaire de (5R,6S) époxy 4,4-diméthyl tétrahyropyr-2-one de formule ($I_A$) :

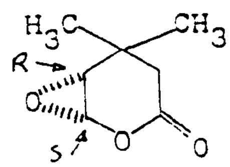

($I_A$)

et de son antipode optique de formule ($I_B$), à savoir le (5S,6R) époxy 4,4-diméthyl tétrahydropyr-2-one : sous formes racémique ou optiquement actives ou sous forme de mélange, R conservant la même signification que précédemment.

2. Procédé selon la revendication 1 pour la préparation des produits de formule (VI) racémiques ou optiquement actifs ainsi que les mélanges de ces différentes formes dans laquelle R a la signification indiquée précédemment, caractérisé en ce que l'on soumet le composé de formule (II) :

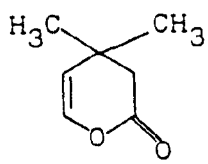

(II)

à l'action d'un agent d'époxydation pour obtenir le composé de formule (I) :

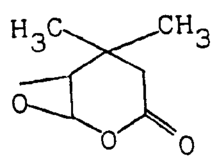

(I)

que l'on sépare éventuellement en chacun de ses énantiomères, produit de formule (I) que l'on soumet à l'action d'un alcool de formule (III) :

R-OH (III)

dans laquelle R a la signification précédente, pour obtenir le composé de formule (IV) correspondant :

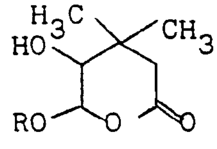

(IV)

que l'on soumet à l'action d'un composé de formule ZX dans laquelle X représente un atome d'halogène et Z représente un radical $R_1A$ dans lequel $R_1$ représente un radical alcoyle renfermant de 1 à 8 atomes de carbone ou un radical aryle éventuellement substitué et A représente un radical $-SO_2-$ ou un radical :

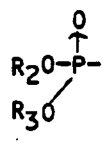

dans laquelle $R_2$ et $R_3$, identiques ou différents, représentent un radical alcoyle renfermant de 1 à 8 atomes de carbone, pour obtenir le composé de formule (V) :

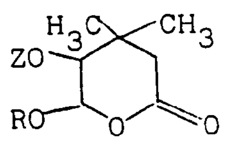

(V)

que l'on soumet à l'action d'un agent basique, pour obtenir le composé de formule (VI) :

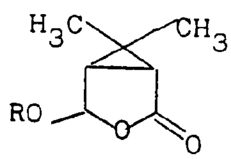

(VI)

R conservant la même signification que précédemment.

3. Procédé selon la revendication 1, pour la préparation des produits de formule (VI) optiquement actifs, caractérisé en ce que l'on soumet le composé de formule (II) :

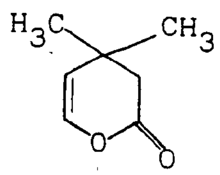

(II)

à l'action d'un agent d'époxydation en présence d'un catalyseur métallique et d'un agent chiral pour obtenir un mélange non équimoléculaire de (5R,6S) époxy 4,4-diméthyl tétrahyropyr-2-one de formule ($I_A$) :

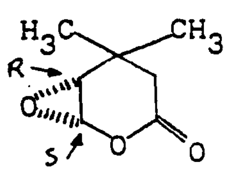

($I_A$)

et de son antipode optique de formule ($I_B$), à savoir le (5S,6R) époxy 4,4-diméthyl tétrahydropyr-2-one :

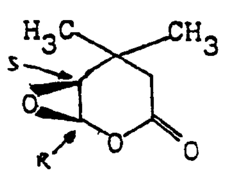

($I_B$)

puis poursuit la synthèse comme indiqué à la revendication 1.

4. Procédé selon la revendication 2 pour la préparation d'un produit de formule (VI) sous forme racémique, caractérisé en ce que l'on soumet le composé de formule (II) à l'action d'un agent d'époxydation pour obtenir le composé de formule (I) sous forme racémique que l'on soumet à l'action d'un composé de formule (III) dans

laquelle R est défini comme précédemment pour obtenir un composé de formule (IV) correspondant sous forme racémique avec les groupements RO et OH en position trans, R étant défini comme précédemment, que l'on soumet à l'action d'un composé de formule ZX dans laquelle Z et X sont définis comme précédemment pour obtenir un composé de formule (V) sous forme racémique avec les groupements ZO et OR en position trans, Z étant défini comme précédemment, que l'on soumet à l'action d'un agent basique pour obtenir le composé de formule (VI) racémique, R conservant la même signification que précédemment.

5. Procédé selon la revendication 2 ou 4, caractérisé en ce que l'agent d'époxydation est soit un peracide minéral ou organique, soit l'eau oxygénée seule ou en présence d'un médiateur ou d'un oxydant capable de transférer un atome d'oxygène, soit un agent biochimique isolé ou au sein d'un milieu plus complexe par oxydation microbiologique.

6. Procédé selon la revendication 5, caractérisé en ce que l'agent d'époxydation est l'acide métachloroperbenzoïque.

7. Procédé selon la revendication 3, caractérisé en ce que le catalyseur métallique est le molybdène hexacarbonyle.

8. Procédé selon la revendication 3 ou 7, caractérisé en ce que l'agent chiral est le L(+) ou le D(-) tartrate de dialkyle.

9. Procédé selon la revendication 8, caractérisé en ce que l'époxydation a lieu en présence de L(+) tartrate de diéthyle en présence de molybdène hexacarbonyle.

10. Procédé selon l'une quelconque des revendications 3 ou 7 à 9, caractérisé en ce que l'agent d'époxydation est l'hydropéroxyde de terbutyle.

11. Procédé selon l'une quelconque des revendications 1 à 10, caractérisé en ce que l'on utilise un alcool de formule (III) dans laquelle R représente un radical méthyle, éthyle ou terbutyle.

12. Procédé selon l'une quelconque de revendications 1 à 11, caractérisé en ce que l'on utilise un composé de formule ZX dans laquelle Z représente un radical $-SO_2CH_3$.

## Patentansprüche

Patentansprüche für die Vertragsstaaten: AT, CH, DE, FR, GB, IT, LI, NL

1. Als neue chemische Produkte in ihren verechiedenen d-, l- und dl-Formen die Verbindung der Formel (I)

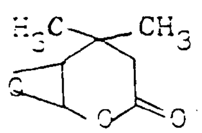

(I)

sowie die Gemische dieser verschiedenen Formen.

2. Als neues chemisches Produkt die Verbindung der Formel (I) in der dl-Form.

3. Verfahren zur Herstellung der Verbindung der Formel (I) gemäß Anspruch 1 oder 2, dadurch gekennzeichnet, daß man der Einwirkung eines Epoxidierungsmittels, ausgewählt unter den Persäuren, wie Peressigsäure, Trifluorperessigsäure, Perphthalsäure, Paranitroperbenzoesäure, Metachlorperbenzoesäure, Monopercamphersäure,oder Wasserstoffperoxid in alkalischer Lösung oder Hexafluoracetonhydroperoxid oder tert.-Butylperoxid oder den biochemischen Epoxidierungsmittel mit einem isolierten Enzym oder auf biochemischem Weg,die Verbindung der Formel (II)

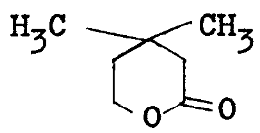

(II)

unterzieht, um die Verbindung der Formel (I) zu erhalten, die man gegebenefalls in ein Jedes ihrer Enantiomeren trennt.

4. Verfahren gemäß Anspruch 3, dadurch gekennzeichnet, daß das Epoxidierungsmittel entweder eine mineralische oder organische Persäure oder Wasserstoffperoxid allein oder in Anwesenheit eines Vermittlers

oder eines Oxidationsmittels, welches zum Transfer eines Sauerstoffatoms befähigt ist, oder ein biochemisches Mittel, isoliert oder in dem Medium eines komplexeren Milieus (mikrobiologische Oxidation) ist.

5. Verfahren gemäß Anspruch 4, dadurch gekennzeichnet, daß das Epoxidierungsmittel Metachlorperbenzoesäure ist.

6. Verfahren zur Herstellung der Verbindung der Formel (I), wie in Anspruch 1 definiert, dadurch gekennzeichnet, daß man die Verbindung der Formel (II)

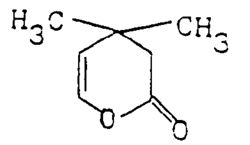

(II)

der Einwirkung eines Epoxidierungsmittels, wie vorstehend definiert, in Gegenwart eines metallischen Katalysators und eines chiralen Mittels unterzieht, um zu einem nichtäqüimolekularen Gemisch von (5R,6S)-Epoxy-4,4-dimethyltetrahydropyr-2-on der Formel ($I_A$)

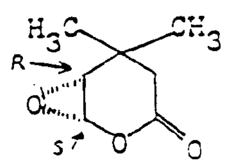

($I_A$)

und seines optischen Antipoden der Formel ($I_B$), nämlich (5S,6R)-Epoxy-4,4-dimethyl-tetrahydropyr-2-on,

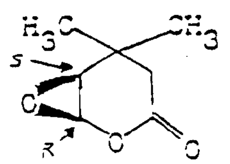

($I_B$)

zu gelangen.

7. Verfahren gemäß Anspruch 6, dadurch gekennzeichnet, daß der metallische Katalysator Molybdänhexacarbonyl ist.

8. Verfahren gemäß Anspruch 6 oder 7, dadurch gekennzeichnet, daß das chirale Mittel das Dialkyl-L(+)- oder -D(-)-tartrat ist.

9. Verfahren gemäß Anspruch 8, dadurch gekennzeichnet, daß die Epoxidation in Anwesenheit von Diethyl-L(+)-tartrat in Anwesenheit von Molybdänhexacarbonyl stattfindet.

10. Verfahren gemäß einem der Ansprüche 6 bis 9, dadurch gekennzeichnet, daß das Epoxidierungsmittel tert.-Butylwasserstoffperoxid ist.

11. Verfahren zur Herstellung der Verbindungen der Formel VI

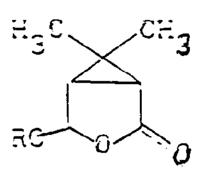

(VI)

worin R einen Methyl-, Ethyl-, n-Propyl-, Isopropyl-, Cyclopropyl-, n-Butyl- oder tert.-Butyl-, Cyclobutyl-, n-Pentyl- oder Cyclopentyl-, Cyclohexyl- oder Menthyl-, 1,1-Dimethylallylrest, gegebenenfalls substituiert durch ein oder mehrere Halogenatome, Gruppen OH oder SH, Gruppen OR′ oder SR′, worin R′ für einen Alkylrest mit 1 bis 8 Kohlenstoffatomen steht, Gruppen $NO_2$ oder

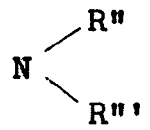,

worin R″ und R‴,die gleich oder voneinander verschieden sind, ein Wasserstoffatom oder einen Alkylrest mit 1 bis 8 Kohlenstoffatomen bedeuten, Gruppen -C≡N oder $PO_4H_2$ oder Gruppen COalc′$_1$, $SO_2$alc′$_2$ oder $SO_3$alc′$_3$, worin alc′$_1$, alc′$_2$ oder alc′$_3$ Reste mit 1 bis 18 Kohlenstoffatomen bedeuten, darstellt oder R einen

Rest $CH_2OCH_3$, $(CH_2)_2OCH_3$, $CH_2CH_2OCH_2CH_3$, 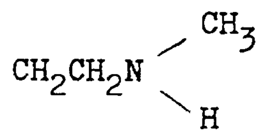,

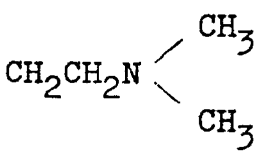, 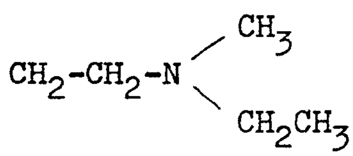

darstellt, oder R einen dadurch gekennzeichnet, daß man die Verbindung der Formel 1, wie in Anspruch 1 definiert, der Einwirkung eines Alkohols der Formel (III)

R-OH (III)

worin R wie vorstehend definiert ist, unterzieht, um die entsprechende Verbindung der Formel (IV)

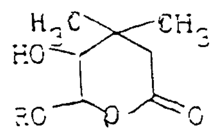

(IV)

zu erhalten, die man der Einwirkung einer Verbindung der Formel ZX unterzieht, worin X ein Halogenatom bedeutet und Z einen Rest $R_1$A, worin $R_1$ einen Alkyrest mit 1 bis 8 Kohlenstoffatomen oder einen gegebenenfalls substituierten Arylrest wiedergibt und A für einen Rest -$SO_2$-oder einen Rest

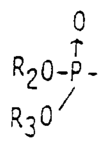

steht, worin $R_2$ und $R_3$, die gleich oder voneinander verschieden sind, einen Alkylrest mit 1 bis 8 Kohlenstoffatomen wiedergeben, darstellt, um zu der Verbindung der Formel (V)

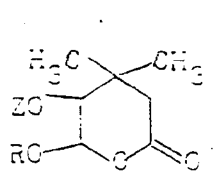

(V)

zu gelangen, welche man der Einwirkung eines basischen Mittels unterzieht, um die Verbindung der Formel (VI)

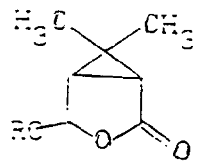

(VI)

zu erhalten, worin R die vorstehend angegebene Bedeutung besitzt.

12. Vewendung gemäß Anspruch 11, dadurch gekennzeichnet, daß

die Verbindung der Formel (I) racemisch ist, die Verbindung der Formel (IV) racemisch ist, wobei die Gruppen RO und OH sich in trans-Stellung befinden und R wie vorstehend definiert ist,

die Verbindung der Formel (V) racemisch ist, wobei die Gruppen ZO und OR sich in trans-Stellung befinden und Z wie vorstehend definiert ist,und

die erhaltene Verbindung der Formel (VI) racemisch ist.

13. Verwendung gemäß Anspruch 12, dadurch gekennzeichnet, daß R für einen Methyl-, Ethyl- oder tert.-Butylrest steht.

14. Verwendung gemäß Anspruch 12 oder 13, dadurch gekennzeichnet, daß Z einen Rest $-SO_2CH_3$ bedeutet.

**Patentansprüche Für folgenden Vertragsstaat: ES**

1. Verfahren zur Herstellung der Verbindungen der Formel (VI)

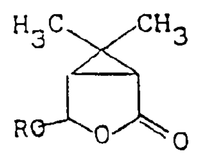

(VI)

in ihren racemischen oder optisch aktiven Formen sowie von deren Gemisch, worin R einen Methyl-, Ethyl-, n-Propyl-, Isopropyl-, Cyclopropyl-, n-Butyl- oder tert.-Butyl-, Cyclobutyl- n-Pentyl- oder Cyclopentyl-, Cyclohexyl- oder Menthyl-, 1,1-Dimethylallyl-Rest, gegebenenfalls substituert durch ein oder mehrere Halogenatome, OH- oder SH-Gruppen, OR′- oder SR′-Gruppen, worin R′ einen Alkylrest mit 1 bis 8 Kohlenstoffatomen wiedergibt, $NO_2$-Gruppen oder Gruppen

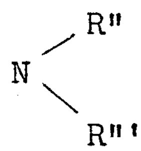

worin R″ und R‴, die gleich oder voneinander verschieden sind, ein Wasserstoffatom oder einen Alkylrest mit 1 bis 8 Kohlenstoffatomen bedeuten, Gruppen -C≡N oder $PO_4H_2$ oder Gruppen COalc′$_1$, $SO_2$alc′$_2$ oder $SO_3$alc′$_3$, worin alc′$_1$, alc′$_2$ oder alc′$_3$ Reste mit 1 bis 18 Kohlenstoffatomen wiedergeben, bedeutet oder R einen Rest $CH_2OCH_3$, $(CH_2)_2OCH_3$, $CH_2CH_2OCH_2CH_3$,

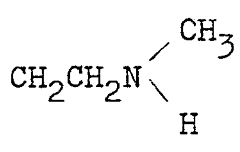 , 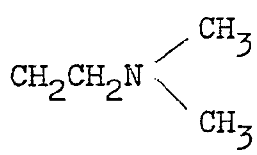 , 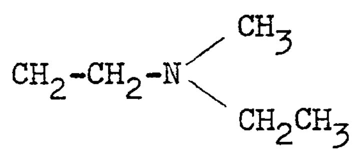

oder R einen Benylrest wiedergibt oder R einen Phenylrest bedeutet, dadurch gekennzeichnet, daß man die Verbindung der Formel (II)

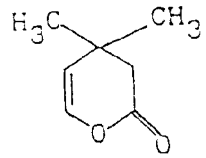

(II)

entweder der Einwirkung eines Epoxidierungsmittels unterzieht, um zu der Verbindung der Formel (I)

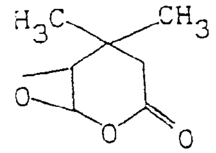

(I)

zu gelangen, die man gegebenenfalls in ein jedes ihrer Enantiomeren trennt, oder der Einwirkung eines Epoxidierungsmittels in Anwesenheit eines metallischen Katalysators und eines Chiralmittels unterzieht, um ein nicht-äquimolekulares Gemisch von (5R,6S)-Epoxy-4,4-dimethyl-tetrahydropyr-2-on der Formel ($I_A$)

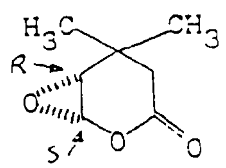

($I_A$)

und seines optischen Antipoden der Formel ($I_B$), nämlich (5S,6R)-Epoxy-4,4-dimethyl-tetrahydropyr-2-on

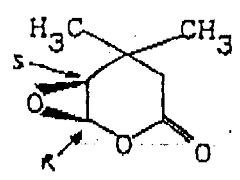

($I_B$)

zu erhalten, das Produkt der Formel (I), ($I_A$) oder ($I_B$) der Einwirkung eines Alkohols der Formel (III)

R-OH (III)

unterzieht, worin R die vorstehende Bedeutung besitzt, um die entsprechende Verbindung der Formel (IV)

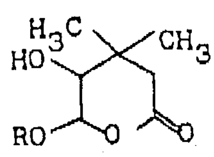

(IV)

in den racemischen oder optisch aktiven Formen oder in Form eines Gemisches zu erhalten, welches man der Einwirkung einer Verbindung der Formel ZX unterzieht, worin X für ein Halogenatom steht und Z einen Rest $R_1A$ darstellt, in dem $R_1$ einen Alkylrest mit 1 bis 8 Kohlenstoffatomen oder einen gegebenenfalls substituierten Arylrest bedeutet und A für einen Rest $-SO_2-$ oder einen Rest

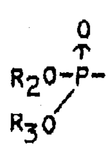

steht, in dem $R_2$ und $R_3$, die gleich oder verschieden sind, einen Alkylrest mit 1 bis 8 Kohlenstoffatomen bedeuten, um die Verbindung der Formel (V)

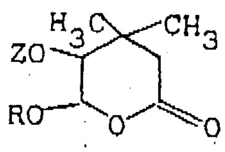

(V)

in den racemischen oder optisch aktiven Formen oder in Form des Gemisches zu erhalten, welches man der Einwirkung eines basischen Mittels unterzieht, um zu der Verbindung der Formel (VI)

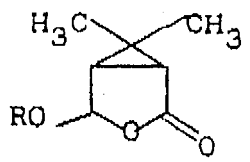

(VI)

in den racemischen oder optisch aktiven Formen oder in Form des Gemisches zu gelangen, wobei R die vorstehend angegebene Bedeutung besitzt.

2. Verfahren gemäß Anspruch 1 zur Herstellung der racemischen oder optisch aktiven Produkte der Formel (VI) sowie der Gemische dieser verschiedenen Formen, worin R die vorstehend angegebene Bedeutung besitzt, dadurch gekennzeichnet, daß man die Verbindung der Formel (II)

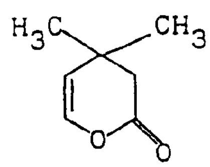

(II)

der Einwirkung eines Epoxidierungsmittels unterzieht, um die Verbindung der Formel (I)

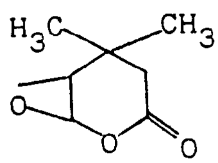

(I)

zu erhalten, die man gegebenenfalls in ein jedes ihrer Enantiomeren trennt, das Produkt der Formel (I) der Einwirkung eines Alkohols der Formel (III)

R-OH (III)

unterzieht, worin R die vorstehende Bedeutung besitzt, um die entsprechende Verbindung der Formel (IV)

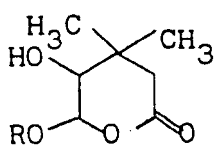

(IV)

zu erhalten, die man der Einwirkung einer Verbindung der Formel ZX unterzieht, worin X ein Halogenatom bedeutet und Z einen Rest $R_1A$ wiedergibt, worin $R_1$ für einen Alkylrest mit 1 bis 8 Kohlenstoffatomen oder einen gegebenenfalls substituierten Arylrest steht und A einen Rest $-SO_2-$ oder

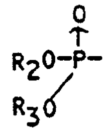

wiedergibt, worin $R_2$ und $R_3$, die gleich oder voneinander verschieden sind, einen Alkylrest mit 1 bis 8 Kohlenstoffatomen bedeuten, um zu der Verbindung der Formel (V)

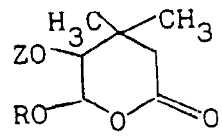

(V)

zu gelangen, die man der Einwirkung eines basischen Mittels unterzieht, um zu der Verbindung der Formel (VI)

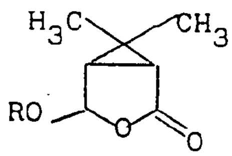

(VI)

zu gelangen, worin R die vorstehend angegebene Bedeutung besitzt.

3. Verfahren gemäß Anspruch 1 zur Herstellung der optisch aktiven Produkte der Formel (VI), dadurch gekennzeichnet, daß man die Verbindung der Formel (II)

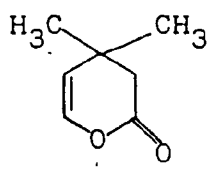

(II)

der Einwirkung eines Epoxidierungsmittels in Gegenwart eines metallischen Katalysators und eines chiralen Mittels unterzieht, um ein nicht-äquimolekulares Gemisch von (5R,6S)-Epoxy-4,4-dimethyltetrahydropyr-2-on der Formel ($I_A$)

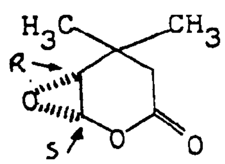

($I_A$)

und ihres optischen Antipoden der Formel (Ig), nämlich (5S,6R)-Epoxy-4,4-dimethyltetrahydropyr-2-on

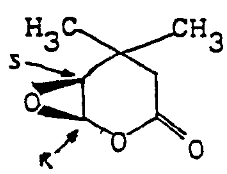

($I_B$)

zu erhalten, wonach man die Synthese wie in Anspruch 1 angegeben fortsetzt.

4. Verfahren gemäß Anspruch 2 zur Herstellung eines Produkts der Formel (VI) in racemischer Form, dadurch gekennzeichnet, daß man die Verbindung der Formel (II) der Einwirkung eines Epoxidierungsmittels unterzieht, um die Verbindung der Formel (I) in racemischer Form zu erhalten, die man der Einwirkung einer Verbindung der Formel (III) unterzieht, worin R wie vorstehend definiert ist, um zu einer entsprechenden Verbindung der Formel (IV) in racemischer Form zu gelangen, in der die Gruppen RO und OH in trans-Position vorliegen, wobei R wie vorstehend definiert ist, welche man der Einwirkung einer Verbindung der Formel ZX unterzieht, worin Z und X wie vorstehend definiert sind, um zu einer Verbindung der Formel (V) in racemischer Form zu gelangen, in der die Gruppen ZO und OR sich in trans-Position befinden, wobei Z wie vorstehend definiert ist, welche man der Einwirkung eines basischen Mittels unterzieht, um die racemische Verbindung der Formel (VI), worin R die vorstehend angegebene Bedeutung besitzt, zu erhalten.

5. Verfahren gemäß Anspruch 2 oder 4, dadurch gekennzeichnet, daß das Epoxidierungsmittel entweder eine mineralische oder organische Persäure oder Wasserstoffperoxid allein oder in Anwesenheit eines Vermittlers oder eines Oxidationsmittels, welches zum Transfer eines Sauerstoffatoms befähigt ist, oder ein biochemisches Mittel, isoliert oder in dem Medium eines komplexeren Milieus durch mikrobiologische Oxidation, ist.

6. Verfahren gemäß Anspruch 5, dadurch gekennzeichnet, daß das Epoxidierungsmittel Metachlorperbenzoesäure ist.

7. Verfahren gemäß Anspruch 3, dadurch gekennzeichnet, daß der metallische Katalysator Molybdänhexacarbonyl ist.

8. Verfahren gemäß Anspruch 3 oder 7, dadurch gekennzeichnet, daß das chirale Mittel das Dialkyl-L(+)- oder -D(-)-tartrat ist.

9. Verfahren gemäß Anspruch 8, dadurch gekennzeichnet, daß die Epoxidierung in Anwesenheit von Diethyl-L(+)-tartrat in Gegenwart von Molybdänhexacarbonyl stattfindet.

10. Verfahren gemäß einem der Ansprüche 3 oder 7 bis 9, dadurch gekennzeichnet, daß das Epoxidierungsmittel das tert.-Butylhydroperoxid ist.

11. Verfahren gemäß einem der Ansprüche 1 bis 10, dadurch gekennzeichnet, daß man einen Alkohol der Formel (III) verwendet, worin R einen Methyl-, Ethyl-oder tert.-Butylrest bedeutet.

12. Verfahren gemäß einem der Ansprüche t bis 11, dadurch gekennzeichnet, daß man eine Verbindung der Formel ZX verwendet, worin Z einen Rest $-SO_2CH_3$ bedeutet.

## Claims

Claims for contracting States: AT, CH, DE, FR, GB, IT, LI, NL

1. As a new chemical product, in its different d, l and dl forms, the compound of formula (I):

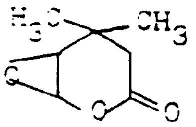

(I)

as well as mixtures of these different forms.

2. As a new chemical product, the compound of formula (I) in its dl form.

3. Preparation process for the compound of formula (I) as defined in claim 1 or 2, characterized in that the compound of formula (II):

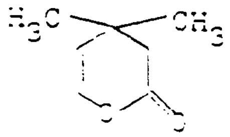

(II)

is subjected to the action of an epoxidation agent, chosen from the peracids such as peracetic acid, trifluoroperacetic acid, perphthalic acid, paranitroperbenzoic acid, metachloroperbenzoic acid, monopercamphoric acid, or hydrogen peroxide in alkaline solution, or hexafluroacetone hydroperoxide or terbutyl hydroperoxide, or biochemical epoxidation agents with an isolated enzyme or by biochemical route, in order to obtain the compound of formula (I) which is optionally separated into each of its enantiomers.

4. Preparation process according to claim 3, characterized in that the epoxidation agent is either a mineral or organic peracid, or hydrogen peroxide by itself or in the presence of a mediator or an oxidizing agent capable of transfering an oxygen atom, or an isolated biochemical agent or in a more complex medium (microbiological oxidation).

5. Process according to claim 4, characterized in that the epoxidation agent is metachloroperbenzoic acid.

6. Preparation process of the compound of formula (I) as defined in claim 1, characterized in that the compound of formula (II):

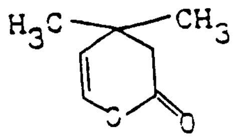

(II)

is subjected to the action of an epoxidation agent as defined previously in the presence of a metal catalyst and a chiral agent in order to obtain a non-equimolecular mixture of (5R,6S) epoxy 4,4-dimethyl tetrahydropyr-2-one of formula ($I_A$):

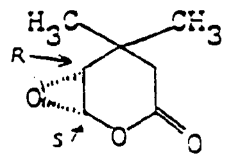

($I_A$)

and its optical antipode of formula ($I_B$), namely (5S,6R) epoxy 4,4-dimethyl tetrahydropyr-2-one:

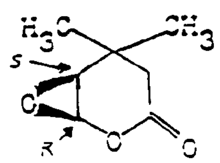

($I_B$)

7. Preparation process according to claim 6, characterized in that the metal catalyst is molybdenum hexacarbonyl.

8. Process according to claim 6 or 7, characterized in that the chiral agent is L(+) or D(-) dialkyl tartrate.

9. Process according to claim 8, characterized in that the epoxidation takes place in the presence of L(+) diethyl tartrate in the presence of molybdenum hexacarbonyl.

10. Process according to any one of claims 6 to 9, characterized in that the epoxidation agent is terbutyl hydroperoxide.

11. Preparation process for the compounds of formula (VI):

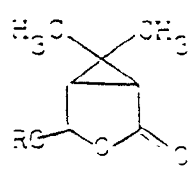

(VI)

in which R represents one of the following radicals: methyl, ethyl, n-propyl, isopropyl, cyclopropyl, n-butyl or terbutyl, cyclobutyl, n-pentyl or cyclopentyl, cyclohexyl or methyl, 1,1-dimethylallyl, optionally substituted by one or more halogen atoms, OH or SH groups, OR′ or SR′ groups in which R′ represents an alkyl radical containing 1 to 8 carbon atoms, $NO_2$ groups or

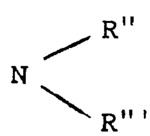

in which R″ and R‴, identical or different, represent a hydrogen atom or an alkyl radical containing 1 to 8 carbon atoms, -C≡N or $PO_4H_2$ groups or COalk′$_1$, $SO_2$alk′$_2$ or $SO_3$′alk$_3$ groups in which alk′$_1$, alk′$_2$ or alk′$_3$ represents radicals with 1 to 18 carbon atoms or R represents a $CH_2OCH_3$, $(CH_2)_2OCH_3$,

$CH_2CH_2OCH_2CH_3$, 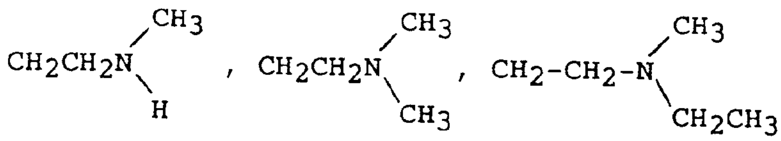

radical or R represents a benzyl radical, or R represents a phenyl radical, characterized in that the compound of formula (I) defined in claim 1 is subjected to the action of an alcohol of formula (III):

R-OH (III)

in which R is defined as previously, in order to obtain the corresponding compound of formula (IV):

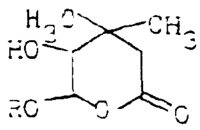

(IV)

which is subjected to the action of a compound of formula ZX in which X represents a halogen atom and Z represents an $R_1A$ radical in which $R_1$ represents an alkyl radical containing 1 to 8 carbon atoms or an optionally substituted aryl radical and A represents an $-SO_2-$ or an

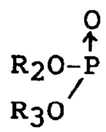

radical, in which $R_2$ and $R_3$, identical or different, represent an alkyl radical containing 1 to 8 carbon atoms, in order to obtain the compound of formula (V):

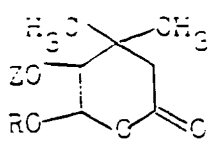

(V)

which is subjected to the action of a basic agent, in order to obtain the compound of formula (VI):

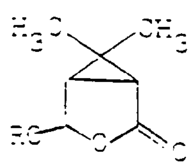

(IV)

R retaining the same meaning as previously.

12. Use according to claim 11, characterized in that:
- the compound of formula (I) is racemic,
- the compound of formula (IV) is racemic with RO and OH groups in <u>trans</u> position, R being defined as previously,
- the compound of formula (V) is racemic with ZO and OR groups in <u>trans</u> position, Z being defined as previously and,
- the compound of formula (VI) obtained is racemic.

13. Use according to claim 12, characterized in that R represents a methyl, ethyl or terbutyl radical.

14. Use according to claim 12 or 13, characterized in that Z represents an $-SO_2CH_3$ radical.

**Claims for the following Contracting State: ES**

1. Preparation process for the compounds of formula (VI):

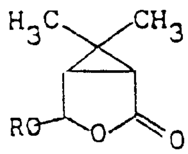

(VI)

in racemic or optically active forms as well as their mixture, in which R represents one of the following radicals: methyl, ethyl, n-propyl, isopropyl, cyclopropyl, n-butyl or terbutyl, cyclobutyl, n-pentyl or cyclopentyl, cyclohexyl or methyl, 1,1-dimethylallyl, optionally substituted by one or more halogen atoms, OH or SH groups, OR′ or SR′ groups in which R′ represents an alkyl radical containing 1 to 8 carbon atoms, $NO_2$ groups or

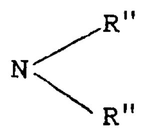

in which R″ and R‴, identical or different, represent a hydrogen atom or an alkyl radical containing 1 to 8 carbon atoms, -C≡N or $PO_4H_2$ groups or COalk′$_1$, $SO_2$alk′$_2$ or $SO_3$′alk$_3$ groups in which alk′$_1$, alk′$_2$ or alk′$_3$ represents radicals with 1 to 18 carbon atoms or R represents a $CH_2OCH_3$, $(CH_2)_2OCH_3$,

$CH_2CH_2OCH_2CH_3$, 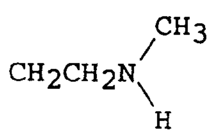 , 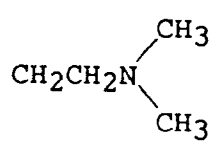 , 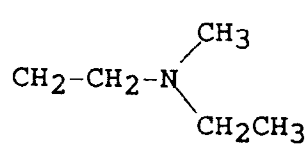

radical or R represents a benzyl radical, or R represents a phenyl radical, characterized in that the compound of formula (II):

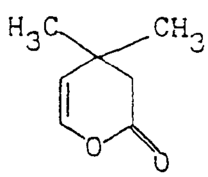

(II)

is subjected either to the action of an epoxidation agent in order to obtain the compound of formula (I):

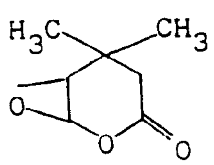

(I)

which is optionally separated into each of its enantiomers, or to the action of an epoxidation agent in the presence of a metal catalyst and a chiral agent in order to obtain a non-equimolecular mixture of (5R,6S) epoxy 4,4-dimethyl tetrahydropyr-2-one of formula (IA):

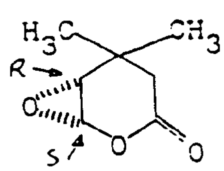

($I_A$)

and its optical antipode of formula ($I_B$), namely (5S,6R) epoxy 4,4-dimethyl tetrahydropyr-2-one:

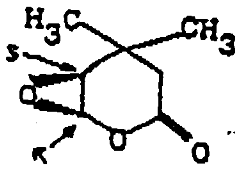

($I_B$)

which product of formula (I), ($I_A$) or ($I_B$) is subjected to the action of an alcohol of formula (III):

R-OH (III)

in which R has the previous meaning, in order to obtain the corresponding compound of formula (IV):

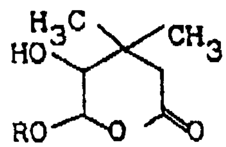

(IV)

in racemic or optically active forms or in the form of a mixture, which is subjected to the action of a compound of formula ZX in which X represents a halogen atom and Z represents an $R_1A$ radical in which $R_1$ represents an alkyl radical containing 1 to 8 carbon atoms or an optionally substituted aryl radical and A represents an $-SO_2-$ radical or a radical:

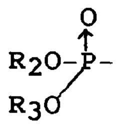

in which $R_2$ and $R_3$, identical or different, represent an alkyl radical containing 1 to 8 carbon atoms, in order to obtain the compound of formula (V):

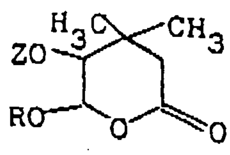

(V)

in racemic or optically active forms or in the form of a mixture, which is subjected to the action of a basic agent in order to obtain the compound of formula (VI):

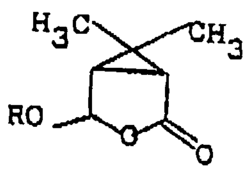

(VI)

in racemic or optically active forms or in the form of a mixture, R retaining the same meaning as previously.

2. Process according to claim 1 for the preparation of the racemic or optically active products of formula (VI) as well as the mixtures of these different forms in which R has the meaning indicated previously, characterized in that the compound of formula (II):

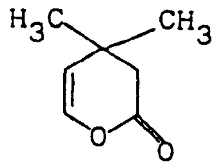

(II)

is subjected to the action of an epoxidation agent in order to obtain the compound of formula (I):

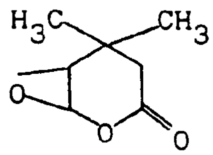

(I)

which is optionally separated into each of its enantiomers, which product of formula (I) is subjected to the action of an alcohol of formula (III):

R-OH (III)

in which R has the previous meaning, in order to obtain the corresponding compound of formula (IV):

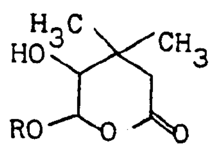

(IV)

which is subjected to the action of a compound of formula ZX in which X represents a halogen atom and Z represents an $R_1A$ radical in which $R_1$ represents an alkyl radical containing 1 to 8 carbon atoms or an optionally substituted aryl radical and A represents an $-SO_2-$ radical or an

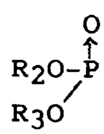

radical, in which $R_2$ and $R_3$, identical or different, represent an alkyl radical containing 1 to 8 carbon atoms, in order to obtain the compound of formula (V):

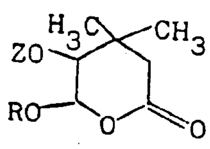

(V)

which is subjected to the action of a basic agent, in order to obtain the compound of formula (VI):

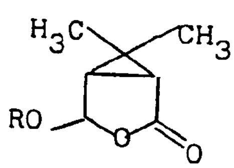

(IV)

R retaining the same meaning as previously.

3. Process according to claim 1, for the preparation of optically active products of formula (VI), characterized in that the compound of formula (II):

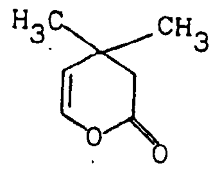

(II)

is subjected to the action of an epoxidation agent in the presence of a metal catalyst and a chiral agent in order to obtain a non-equimolecular mixture of (5R,6S) epoxy 4,4-dimethyl tetrahydropyr-2-one of formula ($I_A$):

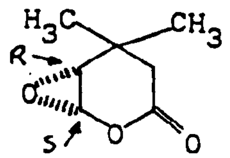

($I_A$)

and its optical antipode of formula ($I_B$), namely (5S,6R) epoxy 4,4-dimethyl tetrahydropyr-2-one:

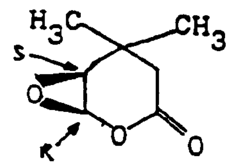

($I_B$)

then the synthesis is continued as indicated in claim 1.

4. Process according to claim 2 for the preparation of a product of formula (VI) in racemic form, characterized in that the compound of formula (II) is subjected to the action of an epoxidation agent in order to obtain the compound of formula (I) in racemic form which is subjected to the action of a compound of formula (III) in which R is defined as previously in order to obtain a corresponding compound of formula (IV) in racemic form with the RO and OH groups in trans position, R being defined as previously, which is subjected to the action of a compound of formula ZX in which Z and X are defined as previously in order to obtain a compound of formula (V) in racemic form with the ZO and OR groups in trans position, Z being defined as previously, which is subjected to the action of a basic agent in order to obtain the racemic compound of formula (VI), R retaining the same meaning as previously.

5. Process according to claim 2 or 4, characterized in that the epoxidation agent is either a mineral or organic peracid, or hydrogen peroxide by itself or in the presence of a mediator or an oxidizing agent capable of transferring an oxygen atom, or an isolated biochemical agent or in a more complex medium by microbiological oxidation.

6. Process according to claim 5, characterized in that the epoxidation agent is metachloroperbenzoic acid.

7. Process according to claim 3, characterized in that the metal catalyst is molybdenum hexacarbonyl.

8. Process according to claim 3 or 7, characterized in that the chiral agent is L(+) or D(-) dialkyl tartrate.

9. Process according to claim 8, characterized in that the epoxidation takes place in the presence of L(+) diethyl tartrate in the presence of molybdenum hexacarbonyl.

10. Process according to any one of claims 3 or 7 to 9, characterized in that the epoxidation agent is terbutyl hydroperoxide.

11. Process according to any one of claims 1 to 10, characterized in that an alcohol of formula (III) is used in which R represents a methyl, ethyl or terbutyl radical.

12. Process according to any one of claims 1 to 11, characterized in that a compound of formula ZX is used in which Z represents an $-SO_2CH_3$ radical.